# EUROPÄISCHE PATENTANMELDUNG

(11) **EP 0 770 359 A1**
(43) Veröffentlichungstag der Anmeldung: **02.05.1997**
(21) Anmeldenummer: 96115723.7
(22) Anmeldetag: 01.10.1996
(51) Int. Cl.: A61B 17/60, A61B 17/80

(54) **Distraktionsvorrichtung für Knochensegmente**

(30) Priorität: 05.10.1995 DE 19537023
(71) Anmelder: Medicon e.G. Chirurgiemechaniker-Genossenschaft, D-78532 Tuttlingen (DE)
(72) Erfinder: Kreidler, Winfried, 78532 Tuttlingen (DE); Wangerin, Konrad, 70192 Stuttgart (DE)
(74) Vertreter: Maser, Jochen, Dipl.-Ing.

(57) **Zusammenfassung**

Die Erfindung betrifft eine Vorrichtung zur Distraktion von mindestens zwei Knochensegmenten mit relativ zueinander verstellbaren Halterungen zur Distraktion von Knochensegmenten im Kiefer-, Gesichts- und Schädelbereich, wobei die Halterung (11) eine zumindest geometrische und zumindest im wesentlichen lineare Längsachse (12), längs der zwei komplementären Hälften (13, 14) durch eine Einstellvorrichtung (16, 18) einstellbar bewegbar sind, und zwar unter Vergrößerung ihres gegenseitigen Abstandes, aufweist, wobei die Halterung (11) wesentlich länger als breit ist, wobei die Hälften (13, 14) durch eine verdrehsichere Führung (17) aneinander längs beweglich geführt sind und an jeder Hälfte (13, 14) zumindest eine flache am Knochen fixierbare Fixiervorrichtung (21, 51) vorgesehen ist, die Schubkräfte und Drehmomente aufnehmen kann und wobei die Einstellvorrichtung (16, 18) an einer Stelle für ein Stellglied zugänglich ist, die zu einer Körperöffnung hingerichtet ist und außerhalb des Endbereichs (34) eine an dem Führungsabschnitt (83) angeordnete Werkzeugaufnahme (87) vorgesehen ist.

## Beschreibung

Die Erfindung betrifft eine Vorrichtung zur Distraktion von Knochensegmenten gemäß dem Oberbegriff des Anspruchs 1. Eine solche Vorrichtung ist in dem deutschen Gebrauchsmuster 94 01 911.8 offenbart. Diese und verwandte Vorrichtungen haben den Nachteil, daß sie von außen an den Knochensegmenten angebracht werden müssen. Dies bedeutet, daß sich außerhalb z.B. des Kiefers und zu beiden Seiten des Kieferastes eine weit abstehende Zahnstangenvorrichtung mit zahlreichen, von außen zugänglichen Schrauben befindet. Fixationsstifte durchqueren die Haut und sind in Knochenlöchern eingelassen. Die Stifte haben eine erhebliche Länge, so daß sie - wenn der Patient die Zahnstangenvorrichtung bewegt - mit großer Kraft mit ihrem freien Ende in den Fixationslöchern herumrühren. Die Haltestifte müssen auch dünn sein, weil die Wunden in der Haut nicht zu groß sein dürfen. Sei es aus kosmetischen Gründen, sei es deshalb, weil große Wunden schlechter zu versorgen sind als kleine Wunden. Dünne Haltestifte neigen zum Verbiegen. Sie werden stark auf Momente beansprucht.

Die bekannte Vorrichtung ist kompliziert in der Bedienung. Sie ist auch teuer in der Herstellung. Je nach Größe und Gestalt des Kiefers sind unterschiedliche Vorrichtungen herzustellen und auf Lager zu halten.

Aufgabe der Erfindung ist es eine Vorrichtung zur Distraktion von Knochensegmenten zu schaffen, die eine Betätigung einer Einstellvorrichtung ohne Verletzung von Gewebe ermöglicht.

Diese Aufgabe wird erfindungsgemäß durch die Merkmale des Anspruchs 1, 18 und 31 gelöst.

Weitere vorteilhafte Ausführungsformen und Weiterbildungen sind in den abhängigen Unteransprüchen angegeben.

Es werden nun bevorzugte Ausführungsbeispiele der Erfindung beschrieben. In der Zeichnung zeigen:
- Fig. 1: die maßstäbliche Draufsicht auf eine Vorrichtung für den rechten Kieferast, gesehen vom Innenraum der Mundhöhle,
- Fig. 2: eine Draufsicht auf eine zweite Vorrichtung, für den linken Kieferast, jedoch von außen gesehen, wobei Fig. 1 und 2 gegenüber der Natur um 200 % vergrößert sind, in ihrem kürzesten Zustand sind und in der Natur dann 62 mm lang sind,
- Fig. 3: die Vorrichtung nach Fig. 2, jedoch im teilweise explodierten Zustand,
- Fig. 4: die Vorrichtung gemäß Fig. 2 oder 3, angebracht an der Innenseite des linken Unterkieferasts,
- Fig. 5: ein Schnitt längs der Linie 5.5 in Fig. 2 in wesentlich vergrößerter Darstellung,
- Fig. 6: ein Radialschnitt durch den rechten Endbereich von Fig. 3
- Fig. 7: einen Schnitt analog der Linie 5.5 in Fig. 2 einer alternative Vorrichtung für den rechten Kieferast,
- Fig. 8: einen teilweisen Radialschnitt durch den rechten Endbereich von Fig. 3 einer vorteilhaften Weiterbildung,
- Fig. 9: einen teilweisen Radialschnitt durch den rechten Endbereich von Fig. 3 einer weiteren vorteilhaften Weiterbildung und
- Fig. 10: eine Draufsicht auf eine weitere Vorrichtung mit einer erfindungsgemäßen Einstellvorrichtung in einer maximalen Distraktionsposition.

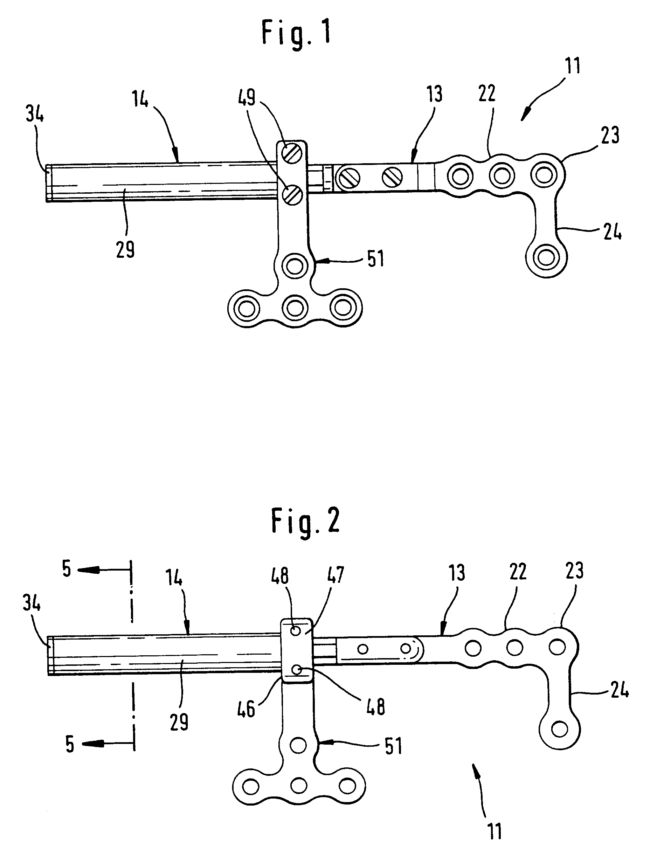

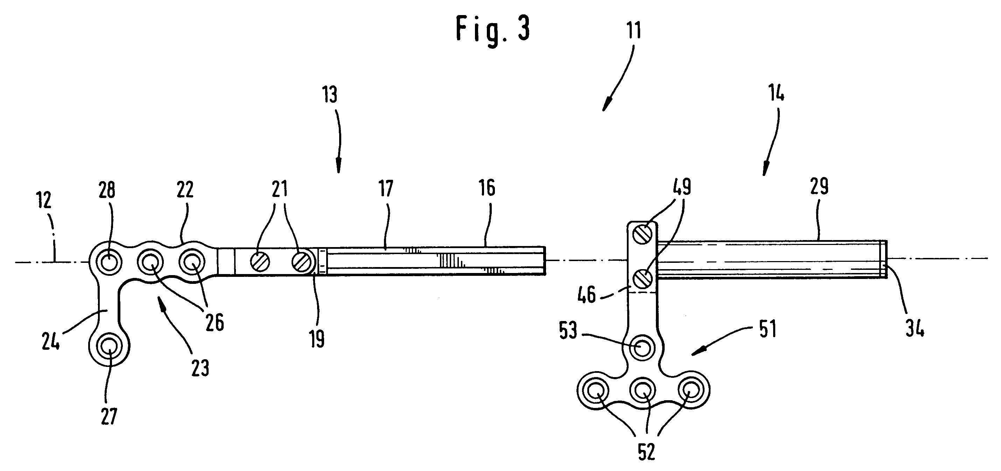

Eine Vorrichtung 11 hat eine geometrische Längsachse 12, längs der ihre beiden Hälften 13 und 14 angeordnet sind. Die Hälfte 13 umfaßt eine Sechskantstange 16, die einen regelmäßigen Außensechskant 17 aufweist, der ein koaxiales Innengewinde 18 besitzt, welches in Fig. 3 nach rechts offen ist. Links hat die Sechskantstange 16 eine Schäftung 19, an welcher mit zwei Schrauben 21 der sich längs der geometrischen Längsachse 12 erstreckende eine Schenke 22 eines Winkelstücks 23 mit seinem Anschraubende befestigt ist. Sein anderer Schenkel 24 ragt senkrecht nach unten. Der eine Schenke hat zwei Befestigungslöcher 26 und der andere Schenke 24 hat ein Befestigungsloch 27 und beide gemeinsam haben ein Befestigungsloch 28. Durch 26, 27 und 28 hindurch können Befestigungsschrauben geschraubt werden, die in den Knochen eingeschraubt werden können. Die Hälfte 14 umfaßt eine koaxiale außenkreiszylindrische Hülse 29, die innen einen koaxialen Innensechskant 31 aufweist, der mit wenig Querspiel den Außensechskant 17 in Längsrichtung führt, und zwar auf einer Strecke, die praktisch seiner gesamten Länge entspricht, wie man aus dem Vergleich zwischen den Figuren 1 und 2 einerseits und der Fig. 3 andererseits entnehmen kann. Die Hülse 29 hat rechts einen ringförmigen Inneneinstich 32. Auf ihrer rechten Stirnfläche 33 sitzt fest eine Kappe 34, die ein koaxiales Zentralloch 36 zum Durchtritt einer Schraubenzieherklinge hat. Ferner hat die Kappe 34 einen Inneneinstich 37, so daß sich zusammen mit dem Inneneinstich 32 ein kreiszylindrischer Innenraum 38 ergibt, wenn die Kappe 34 fest mit dem rechten Endbereich der Hülse 29 verbunden ist. Im Innenraum 38 kann sich der koaxiale Kopf 41 einer Gewindeschraube 42 in Umfangsrichtung wegen des vorhandenen Spiels frei drehen. In Längsrichtung ist der Kopf 41 mit geringem Spiel im Innenraum 38 gehalten und kann nicht nach außen fallen, weil das Zentralloch 36 einen kleineren Durchmesser als der Kopf 41 hat. Die Gewindeschraube 42 hat einen Schaft 43, welcher ein Außengewinde 44 hat, welches mit dem Innengewinde 18 kämmt. Dreht man den Kopf 41 in die eine Richtung, bewegt sich die Sechskantstange 16 nach außen. In der anderen Richtung bewegt sie sich nach innen.

Im linken Endbereich der Hülse 29 befindet sich ein Metallklötzchen 46, das fest mit der Hülse 29 verbunden ist, in einem Zentralloch die Hindurchbewegung der Sechskantstange 16 gestattet und zu beiden Seiten je eine Abflachung 47 aufweist. Durch das Metallklötzchen 48 sind zwei Durchgangs-Gewindelöcher 48 gebohrt. Auf die eine jeweils dienliche Abflachung 47 ist mit zwei Schrauben 49 das Befestigungsende eines T-Stücks 51 geschraubt, das ebenfalls T-förmig angeordnete Befestigungslöcher 52 und ein in Querrichtung angeordnetes Befestigungsloch 53 aufweist, durch die hindurch Knochenschrauben gedreht werden können.

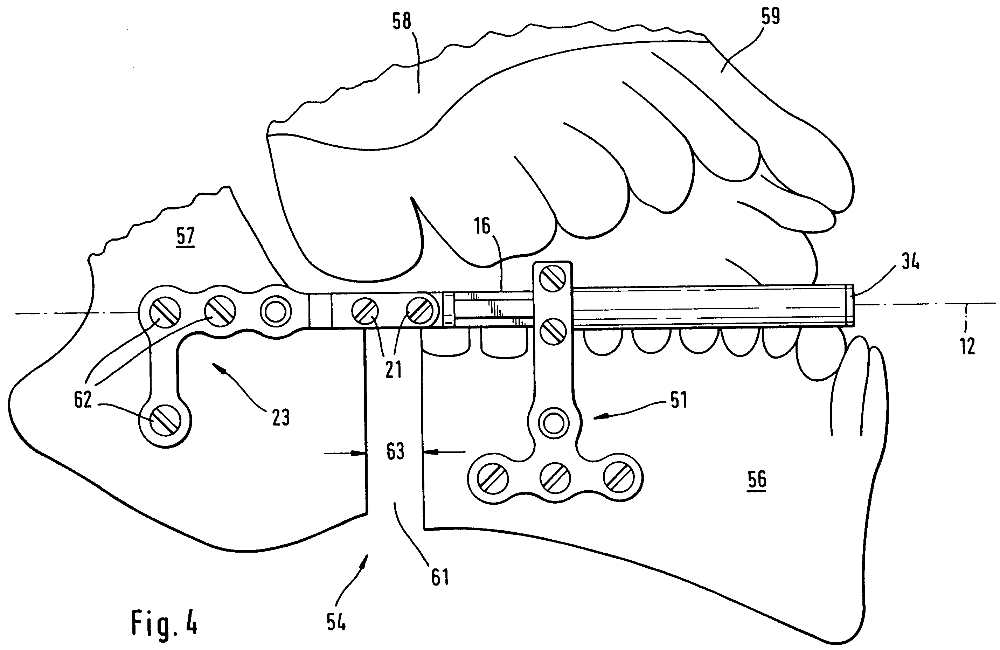

Gemäß Fig. 4 hat ein linker Unterkiefer 54 einen horizontalen Ast 56 und einen aufsteigenden Ast 57. Auch der Oberkiefer 58 mit seinen Zähnen 59 ist zur Orientierung angedeutet. Der Ast 56 war durch einen Schnitt 61 getrennt worden, und links davon wurde das Winkelstück 23 mit Schrauben 62 befestigt, so daß Schubkräfte und Momentkräfte aufgenommen werden konnten. Am rechten Teil des Astes 56 wurde das T-Stück 51 befestigt, so daß Schubkräfte und Momentkräfte übertragen werden konnten. Das Winkelstück 23 und das T-Stück 51 wurden direkt auf dem Knochen befestigt. Die Sechskantstange 16 ist schon fast zur Hälfte ausgefahren. Die Kappe 34 befindet sich an einer Stelle innerhalb und hinter dem linken Mundwinkel, so daß man bei geschlossenem Mund von der Vorrichtung 11 überhaupt nichts sieht.

Innerhalb der Vorrichtung 11 zum Beispiel zwischen dem Kopf 41 und dem Einstich 32 kann man auch eine Druckfeder als Kraftspeicher vorsehen, so daß die Dilatationskraft über einen längeren Zeitraum abgegeben wird.

Der Innenraum der Hülse 29 würde alternativ auch genügend Platz für einen elektrischen Mikromotor aufweisen, der als Niedervoltmotor sich langsam drehen würde und so die Sechskantstange 16 oder ein äquivalentes Teil aus der Hülse 29 hinausschieben würde.

Es wäre auch Platz für einen hydraulischen oder pneumatischen Kraftspeicher, der seine Kraft ebenfalls über einen längeren Zeitraum dosiert abgeben würde, wobei man z.B. bei kompressivem Fluid den Druck im Kraftspeicher einmal aufbaut und dieser dann über einen längeren Zeitraum wirkt.

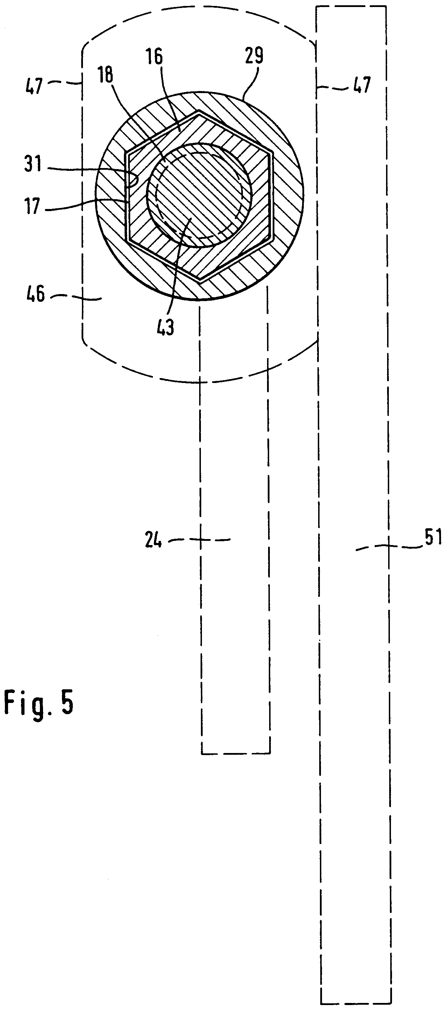

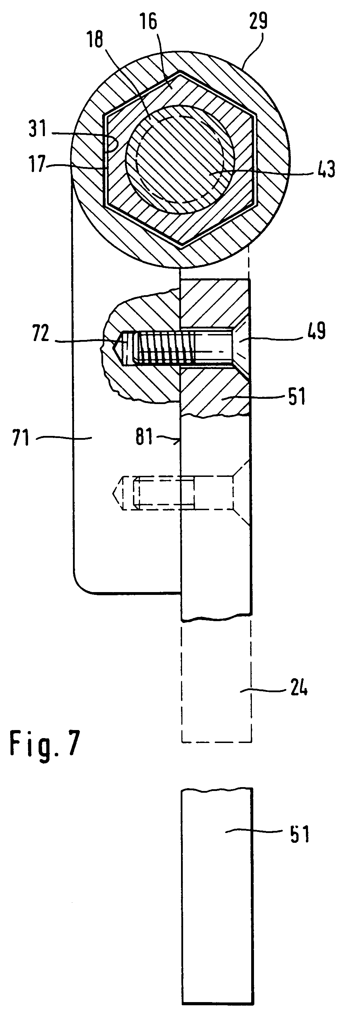

In Fig. 7 ist eine alternative Ausführungform einer Vorrichtung 11 dargestellt. Im Unterschied zu der in den Figuren 1 bis 6 dargestellten Vorrichtung 11 ist anstelle der Metallklötzchen 46 in Fig. 5 eine Aufnahme 71 vorgesehen, die unmittelbar an der Hülse 29 angeformt ist. Diese Aufnahme 71 ist als ein Arm, der im wesentlichen senkrecht zur Längsachse 12 der Hülse 29 verläuft, ausgebildet. Eine Aufnahmefläche 81 zur Anordnung der Platte 51 ist dabei konstruktiv derart angeordnet, daß die aufgenommene Platte 51 im wesentlichen in derselben Ebene des Schenkels 24, der über ein Winkelstück 23 und einen Schenkel 22 an der Hälfte 13 angeordnet ist, liegt. Die Platte 51 ist ebenfalls durch zwei Schrauben 49, die vorteilhafterweise als Senkkopfschrauben ausgebildet sind, an Bohrungen 72 zu der Aufnahme 71 angeordnet.

Diese Anordnung weist den Vorteil auf, daß eine erhebliche Reduzierung in der Bauteilgröße gegeben sein kann. Darüber hinaus weist diese Anordnung den Vorteil auf, daß ein einfaches Handling durch die in einer Ebene liegende Platte 51 und des Schenkels 24 gegeben ist. Dadurch kann eine Verformung zumindest der Platte 51 geringer ausgebildet sein, wodurch eine spätere Materialermüdung eintritt, um die Vorrichtung 11 an einem Kieferast anzubringen. Darüber hinaus ist eine derartige Ausführungsform der Vorrichtung für das Implantat schonender.

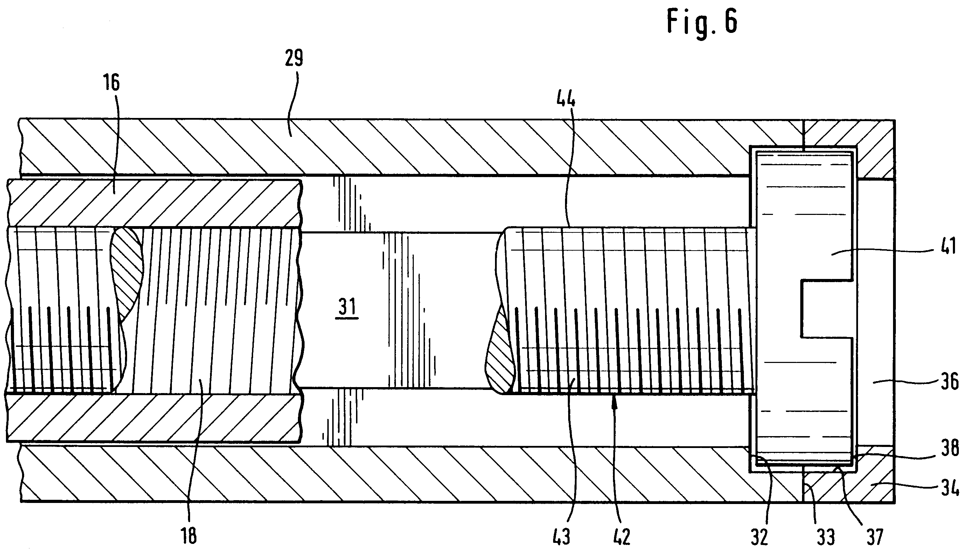

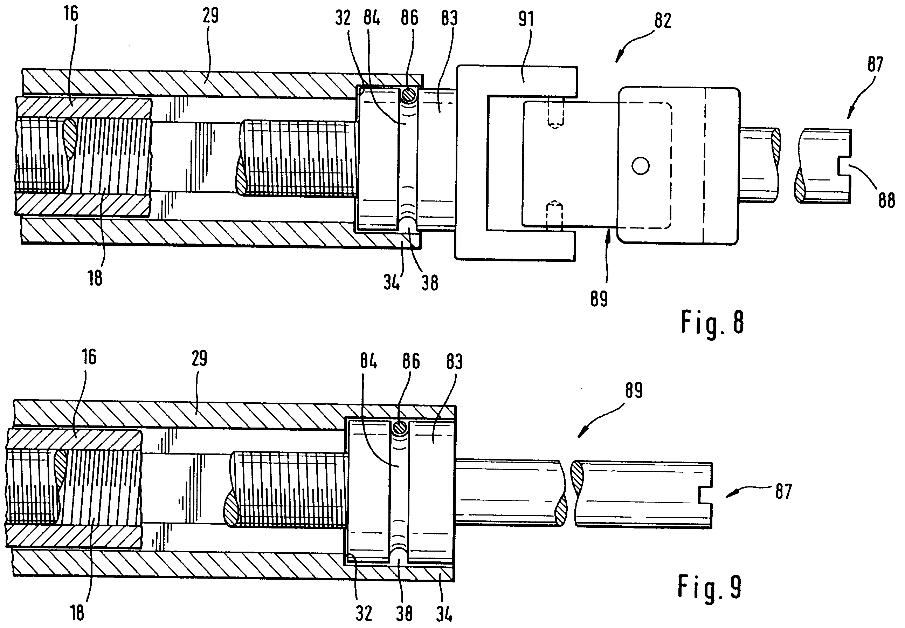

Die übrigen Bauteile dieser Vorrichtung 11 entsprechen der in Fig. 1 bis 6 beschriebenen Distraktionsvorrichtung 11. Ebenso kann eine in den Figuren 8 und 9 beschriebenen Einstellvorrichtung an der in Fig. 6 beschriebenen Vorrichtung 11 angeordnet sein.

In Fig. 8 ist eine alternative Ausführungsform einer Vorrichtung 11 gemäß den Figuren 1 bis 6 dargestellt. Diese Vorrichtung 11 ist bevorzugt zur Anordnung am aufsteigenden Unterkieferast einsetzbar. Diese Vorrichtung 11 bietet jedoch auch am horizontalen Ast 56, 57 bei entsprechend ungünstigen anatomischen Verhältnissen Vorteile.

Die Vorrichtung 11 weist zur Einstellung des Abstandes zwischen einem hinteren und vorderen Abschnitt eines Astes des Unterkiefers eine Einstellvorrichtung 82 auf. Diese weist einen sich an das Innengewinde 18 anschließenden Führungsabschnitt 83 auf, der in einem Endbereich 34 der Hülse 29 geführt ist. Der Führungsabschnitt 83 weist einen halbkreisförmigen Einstich 84 auf, zu dem tangential ein Stift 86 im Endbereich 34 angeordnet ist, um den Führungsabschnitt 83 in dem durch den Inneneinstich 32 gebildeten Innenraum 38 anzuordnen und das Innengewinde 18 axial zu sichern. Alternativ kann auch vorgesehen sein, daß ein rechteckiger Einstich 84 an dem Führungsabschnitt 83 vorgesehen ist. Des weiteren können alternative Ausbildungen zur axialen Sicherung vorgesehen sein. Der Führungsabschnitt 83 ragt aus dem freien Endbereich 34 heraus und weist an seinem freien Ende eine Werkzeugaufnahme 87 auf. Zur Aufnahme von einem Schraubendreher ist ein Schlitz 88 vorgesehen, der die Werkzeugaufnahme 87 bildet. Alternativ kann hierbei vorgesehen sein, daß die Werkzeugaufnahme 87 als Kreuzschlitz, Innen- oder Außen-Sechskant oder für weitere Werkzeuge ausgebildet sein kann.

Zwischen dem Führungsabschnitt 83 und der Werkzeugaufnahme 87 ist eine Gelenkverbindung 89 vorgesehen, die ermöglicht, daß beim Anbringen der Vorrichtung 11 an einem aufsteigenden Unterkieferast eine Einstellung der Vorrichtung 11 ermöglicht ist, bei der das Werkzeug durch die Mundhöhle eingeführt werden kann. Dadurch kann vermieden werden, daß bei der Anwendung der bisherigen Vorrichtung am aufsteigenden Ast eine Inzision in der Wange zum Einführen eines Schraubendrehers erforderlich ist. Die Gelenkverbindung 89 ist zumindest um wenige Grade aus der geometrischen Längsachse 12 der Vorrichtung 11 heraus bewegbar. Diese kann beispielsweise als Kugelgelenk, Kardangelenk oder dergleichen ausgebildet sein. Die Gelenkverbindung 89 ist vorteilhafterweise sehr klein ausgebildet, so daß die an dem Führungsabschnitt 83 angeordnete Gabel 91 zur Aufnahme der Gelenkverbindung 89 im Durchmesser im wesentlichen der Hülse 29 der Hälfte 14 entspricht oder nur geringfügig größer ausgebildet ist.

In Fig. 9 ist eine alternative Ausführungsform einer Einstellvorrichtung 82 zu der in Fig. 8 dargestellten Vorrichtung dargestellt. Die Gelenkverbindung 89 ist als flexible Welle ausgebildet. Diese kann beispielsweise als eine Drahtspirale, ein Drahtseil, das gegen die Drehrichtung gedrillt ist, oder als Federdraht ausgebildet sein. Vorteilhafterweise kann um die Drahtspirale oder das Drahtseil eine Hülse angebracht sein, um der flexiblen Welle eine gewisse Drehsteifigkeit zu geben. Darüber hinaus können weitere Ausführungsformen zur Ausbildung einer flexiblen Welle vorgesehen sein, die beispielsweise aus flexiblen Materialkombinationen bestehen können, um das auf die Werkzeugaufnahme 87 über das Werkzeug aufgebrachte Drehmoment auf die Einstellvorrichtung 82 zu übertragen, so daß die erste Hälfte 13 zu der zweiten Hälfte 14 bewegbar ist. Der Endbereich 34 der Hülse 29 kann vorteilhafterweise den gesamten Führungsabschnitt 83 umgreifen, wodurch die Führung und die Kraftaufnahme während des Einstellvorgangs erhöht werden kann.

Die Vorrichtung 11 kann alternativ zu den in Fig. 8 und 9 beschriebenen Einstellvorrichtungen 82 auch durch ein Getriebe gegeben sein. Dabei können Kegelräder in Einsatz kommen, die in gewissen Gradabstufungen zu der geometrischen Längsachse 12 der Vorrichtung 11 die Werkzeugaufnahme 87 anordnen.

Des weiteren kann vorgesehen sein, daß unmittelbar an dem Führungsabschnitt 83 die Werkzeugaufnahme 87 angeordnet ist, so daß zum Beispiel die Gelenkverbindung 89 an einem Werkzeug angeordnet ist, die auf der Werkzeugaufnahme 87, die fest zu dem Innengewinde 18 angeordnet ist, aufsteckbar sein kann.

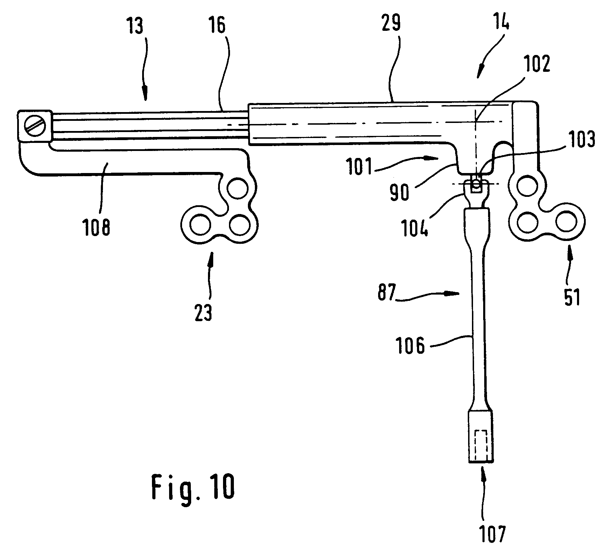

In Figur 10 ist eine weitere alternative Ausführungsform einer Distraktionsvorrichtung dargestellt, bei der die Einstellvorrichtung 101 zwischen den Fixiervorrichtungen 23, 51 angeordnet ist. Die Einstelleinrichtung 101 weist eine in der Hülse 29 der ersten Hälfte 14 angeordnete Gelenkverbindung 102 auf. Diese Gelenkverbindung 102 greift mit einem Ausgang an der Hälfte 13 an, wobei der zweite Ausgang der Gelenkverbindung 102 als Antriebsstange 103 ausgebildet ist. Die Hülse 29 weist zur Fixierung und Führung der Antriebsstange 103 einen Führungsabschnitt 90 auf, der an die Hülse 29 angeformt ist. Die Gelenkverbindung 102 ist als Kegelradgetriebe ausgebildet. Alternativ können weitere Getriebearten, wie beispielsweise Schneckenradgetriebe, eingesetzt werden, die die Funktion erfüllen, daß eine Antriebsachse einer Antriebsstange 103 abweichend von einer Längsachse 12 der Vorrichtung 11 angeordnet ist. Die Übersetzungsverhältnisse der verwendeten Gelenkverbindung 102 können auf die jeweiligen Anwendungsfälle angepaßt werden, so daß eine einfache und präzise Distraktion der Knochen durch die Hälfte 13 und 14 gegeben sein kann.

Außerhalb des Führungsabschnittes 90 ist ein Gelenk 104 angeordnet, das eine Verlängerung 107 aufnimmt. An dem freien Ende der Verlängerung 106 ist ein Werkzeugschlüssel (nicht dargestellt) an einer Aufnahme 107 anbringbar. Dadurch kann eine Betätigung der Gelenkverbindung 102 in unterschiedlichen Winkellagen zu der Antriebsstange 103 gegeben sein.

Es kann auch vorgesehen sein, daß die Verlängerung 106 von dem Gelenk 104 abnehmbar ausgebildet ist.

Eine weitere alternative Ausführungsform der Erfindung weist eine Antriebsstange 103 mit einer Werkzeugaufnahme 107 auf, die an dem der Gelenkverbindung 102 gegenüberliegenden Ende vorgesehen ist. Die Werkzeugaufnahme 107 ist derart ausgebildet, daß diese gleichzeitig als Aufnahme für ein Gelenk 104 oder eine Verlängerung 106 ausgebildet ist.

Somit kann anwendungsspezifisch das Gelenk 104 und/oder die Verlängerung 106 zur Antriebsstange 103 positionierbar sein.

Die Ausbildung des Führungsabschnittes 90 an der Hülse 29 ist gemäß dem Ausführungsbeispiel in der Ebene der Fixiervorrichtungen 23, 51 angeordnet. Alternativ kann vorgesehen sein, daß für bestimmte Anwendungsfälle der Führungsabschnitt 90 abweichend von der in Figur 10 dargestellten Anordnung über den Umfang verteilt an der Hülse 29 angeordnet sein kann. So kann beispielsweise der Führungsabschnitt um 180° um die Längsachse 12 gedreht an der Hülse 29 angeformt sein.

Ebenso können beispielsweise analoge Ausführungsformen gemäß der Fig. 1 bis 3, 6, 8 und 9 am zweiten Ausgang der Gelenkverbindung 102 ausgebildet sein.

Die Hälfte 13 weist einen n-kantigen Außenquerschnitt auf, der vorzugweise als Außenvierkant 16 ausgebildet ist und im wesentlichen der Länge der Hälfte 14 entspricht, so daß dieser vollständig eintauchen kann. Am freien Ende der Hälfte 13 ist ein parallel zum Außenvierkant 16 angeordnetes Halteelement 108 angeordnet, welches die Fixiervorrichtung 23 trägt. Durch diese Anordnung ist ermöglicht, daß beim Eintauchen des Außenvierkants 16 der Hälfte 13 in die Hülse 29 der Hälfte 14 die Fixiervorrichtungen 23 und 51 mit Ausnahme des Bereiches für die Gelenkverbindung 102 bzw. des Führungsabschnitts 90 eng aneinander liegend positioniert werden können.

Die Integration der Gelenkverbindung 102 in die Hülse 29 kann ebenso bei der Ausführungsform gemäß Figur 1 bis 3 vorgesehen sein und ist auf weitere spezielle Ausführungsformen der Halterungen übertragbar.

Des weiteren sind die vorteilhaften Ausgestaltungen und Anordnungen der Figuren 1 bis 9 in beliebiger Kombination miteinander ebenso auf eine Ausführungsform gemäß Figur 10 übertragbar.

## Patentansprüche

1. Vorrichtung zur Distraktion von mindestens zwei Knochensegmenten mit relativ zueinander verstellbaren Halterungen zur Distraktion von Knochensegmenten im Kiefer-, Gesichts- und Schädelbereich, bei der
- die Halterung (11) eine zumindest geometrische und zumindest im wesentlichen lineare Längsachse (12) hat, längs der zwei komplementäre Hälften (13, 14) durch eine Einstellvorrichtung (41, 43, 16, 18; 82) einstellbar bewegbar sind, und zwar unter Vergrößerung ihres gegenseitigen Abstandes (63)
- die Halterung (11) wesentlich länger als breit ist,
- die Hälften durch eine verdrehsichere Führung (17, 31) aneinander längsbeweglich geführt sind,
- an jeder Hälfte (13, 14) zumindest je eine flache, am Knochen (56, 57) fixierbare Fixiervorrichtung (23, 51) vorgesehen ist, die Schubkräfte und Drehmomente aufnehmen kann,
- die Einstellvorrichtung (41, 43, 16, 18; 82) an einer Stelle (36) für ein Stellglied (42) zugänglich ist, die im wesentlichen zu einer Körperöffnung hin gerichtet ist, dadurch gekennzeichnet, daß die Einstellvorrichtung (82) mit ihrer einen Hälfte (14) eine Hülse (29) umfaßt, deren freien Endbereich (34) zumindest teilweise offen ist und daß in diesem Endbereich (34) ein Führungsabschnitt (83) drehbar gelagert ist und außerhalb des Endbereichs (34) eine an dem Führungsabschnitt (83) angeordnete Werkzeugaufnahme (87) vorgesehen ist.

2. Vorrichtung nach Anspruch 1, dadurch gekennzeichnet, daß die Werkzeugaufnahme (87) in einer von der Längsachse (12) abweichenden Winkellage positionierbar ist.

3. Vorrichtung nach Anspruch 1 oder 2, dadurch gekennzeichnet, daß zwischen dem Führungsabschnitt (83) und der Werkzeugaufnahme (87) eine Gelenkverbindung (89) vorgesehen ist.

4. Vorrichtung nach Anspruch 3, dadurch gekennzeichnet, daß die Gelenkverbindung (89) als ein Kardangelenk oder Kugelgelenk ausgebildet ist.

5. Vorrichtung nach Anspruch 3, dadurch gekennzeichnet, daß die Gelenkverbindung (89) als eine flexible Welle ausgebildet ist.

6. Vorrichtung nach Anspruch 3, dadurch gekennzeichnet, daß die Gelenkverbindung (89) als Winkelgetriebe ausgebildet ist.

7. Vorrichtung nach Anspruch 1, dadurch gekennzeichnet, daß die eine Hälfte (14) als Hülse (29) ausgebildet ist, die zur Aufnahme der Fixiervorrichtung (51) eine an die Hülse (29) angeformte Aufnahme (71) aufweist.

8. Vorrichtung nach Anspruch 7, dadurch gekennzeichnet, daß die Aufnahme (71) im wesentlichen rechtwinklig zu der Längsachse der Hülse (29) angeordnet ist.

9. Vorrichtung nach Anspruch 7 oder 8, dadurch gekennzeichnet, daß die Aufnahme (71) ein Befestigungselement (72), vorzugsweise eine Gewindebohrung aufweist, an dem die Fixiervorrichtung (51) befestigbar ist.

10. Vorrichtung nach einem der Ansprüche 7 bis 9, dadurch gekennzeichnet, daß die Aufnahme (71) an der Hülse (29) derart angeformt ist, daß die Fixiervorrichtungen (23, 51) in einer gemeinsamen Ebene anordenbar sind.

11. Vorrichtung zur Distraktion von mindestens zwei Knochensegmenten mit relativ zueinander verstellbaren Halterungen zur Distraktion von Knochensegmenten im Kiefer-, Gesichts- und Schädelbereich, bei der
- die Halterung (11) eine zumindest geometrische und zumindest im wesentlichen lineare Längsachse (12) hat, längs der zwei komplementäre Hälften (13, 14) durch eine Einstellvorrichtung (41, 43, 16, 18; 101) einstellbar bewegbar sind, und zwar unter Vergrößerung ihres gegenseitigen Abstandes (63)
- die Halterung (11) wesentlich länger als breit ist,
- die Hälften durch eine verdrehsichere Führung (17, 31) aneinander längsbeweglich geführt sind,
- an jeder Hälfte (13, 14) zumindest je eine flache, am Knochen (56, 57) fixierbare Fixiervorrichtung (23, 51) vorgesehen ist, die Schubkräfte und Drehmomente aufnehmen kann,
- die Einstellvorrichtung (41, 43, 16, 18; 101) an einer Stelle (36) für ein Stellglied (42) zugänglich ist, die im wesentlichen zu einer Körperöffnung hin gerichtet ist,
dadurch gekennzeichnet, daß eine zwischen den Fixiervorrichtungen (23, 51) angeordnete eine Werkzeugaufnahme (87) aufweisende Einstellvorrichtung (101) vorgesehen ist.

12. Vorrichtung nach Anspruch 11, dadurch gekennzeichnet, daß die Einstellvorrichtung (101) als eine in der Hülse (29) angeordnete Gelenkverbindung (102) ausgebildet ist.

13. Vorrichtung nach Anspruch 11 oder 12, dadurch gekennzeichnet, daß die Einstellvorrichtung (101) einen von der geometrischen Längsachse (12) abweichenden Führungsabschnitt (90) zur Führung einer Antriebsstange (103) der Gelenkverbindung (102) aufweist.

14. Vorrichtung nach einem der Ansprüche 11 bis 13, dadurch gekennzeichnet, daß die Gelenkverbindung (102) als ein Winkelgetriebe, vorzugsweise Kegelrad- oder Schneckenradgetriebe, ausgebildet ist.

15. Vorrichtung nach Anspruch 12, dadurch gekennzeichnet, daß außerhalb des Führungsabschnitts (90) die Antriebsstange (103) ein Gelenk (104) aufweist.

16. Vorrichtung nach Anspruch 15, dadurch gekennzeichnet, daß an dem Gelenk (104) eine Verlängerung (106) mit einer Werkzeugaufnahme (107) angeordnet ist.

17. Vorrichtung nach Anspruch 15, dadurch gekennzeichnet, daß die Antriebsstange (103), das Gelenk (104) und die Verlängerung (106) einstückig ausgebildet sind.

18. Vorrichtung nach Anspruch 15, dadurch gekennzeichnet, daß das Gelenk (104) und die Verlängerung (106) lösbar zur Antriebsstange (103) angeordnet sind.

19. Vorrichtung nach Anspruch 13, dadurch gekennzeichnet, daß der Führungsabschnitt (90) in der Ebene der Fixiervorrichtungen (23, 51) angeordnet ist.

20. Vorrichtung nach Anspruch 11, dadurch gekennzeichnet, daß die Hälfte (13) einen Abschnitt (108) aufweist, der im wesentlichen parallel zu dieser verläuft und an dessen einen Ende eine Fixiervorrichtung aufnimmt.

21. Vorrichtung nach Anspruch 1, dadurch gekennzeichnet, daß eine linke (Fig. 2, 3) und eine rechte (Fig. 1) Vorrichtung für den linken und den rechten Kieferast vorgesehen ist.

22. Vorrichtung nach Anspruch 1, dadurch gekennzeichnet, daß die linke und rechte Vorrichtung für den jeweiligen horizontalen (56) und aufsteigenden (57) Kieferast vorgesehen ist.
